(19) Europäisches Patentamt
European Patent Office
Office européen des brevets

(11) **EP 4 742 163 A1**

(12) **EUROPEAN PATENT APPLICATION**
published in accordance with Art. 153(4) EPC

(43) Date of publication:
**13.05.2026   Bulletin 2026/20**

(21) Application number: 23944245.2

(22) Date of filing: **20.12.2023**

(51) International Patent Classification (IPC):
**G06T 11/00** (2026.01)     **G06T 3/00** (2024.01)
**A61B 6/00** (2024.01)

(52) Cooperative Patent Classification (CPC):
**A61B 6/00; G06T 3/00; G06T 11/00**

(86) International application number:
**PCT/CN2023/140100**

(87) International publication number:
**WO 2025/007524 (09.01.2025 Gazette 2025/02)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  06.07.2023   CN 202310820426
01.08.2023   CN 202310958795

(71) Applicant: **Yofo (Hefei) Medical Technology Co.,
Ltd.**
**Hefei, Anhui 230088 (CN)**

(72) Inventors:
• **YU, Wenrui**
**Hefei, Anhui 230088 (CN)**

• **MA, Junqi**
**Hefei, Anhui 230088 (CN)**
• **XU, Ran**
**Hefei, Anhui 230088 (CN)**
• **LIU, Zhe**
**Hefei, Anhui 230088 (CN)**
• **CAI, Yunyan**
**Hefei, Anhui 230088 (CN)**
• **GUAN, Xianjin**
**Hefei, Anhui 230088 (CN)**

(74) Representative: **Meyer-Dulheuer MD Legal
Patentanwälte PartG mbB
Hanauer Landstr. 287-289
60314 Frankfurt am Main (DE)**

(54) **CT IMAGING METHOD AND APPARATUS, AND CBCT DEVICE**

(57)    The present disclosure provides a CT imaging method, an apparatus, and a CBCT device. The method comprises: performing several scans on an object to be scanned and obtaining one set of projection images respectively for each scan of the several scans; performing a backprojection weighting on several projection images at the same scan angle after obtaining at least two sets of projection images, or performing the backprojection weighting on the projection images obtained at a current scan angle during each scan, to obtain weighted projection data; and obtaining volume data of the object based on the weighted projection data.

M10

S100
performing several scans on an object to be scanned and obtaining one set of projection images respectively for each scan of the several scans, the several scans comprise N circular scans and M helical scans with N≥2 and N>M≥1

S200
performing a backprojection weighting on several projection images at the same scan angle after obtaining at least two sets of projection images, or performing the backprojection weighting on the projection images obtained at a current scan angle during each scan, to obtain weighted projection data

S300
obtaining volume data of the object based on the weighted projection data

Fig. 1

## Description

TECHNICAL FIELD

The present disclosure relates to a CT imaging method, an apparatus, and a CBCT device.

BACKGROUND

During a measurement of a CBCT (Cone Beam CT) device, an X-ray source and a detector are controlled to rotate around a patient's head to complete scanning. To increase a field of view in an axial direction, a dual-source single-detector system can be employed to expand the axial field of view. A dual-source single-detector system having two X-ray sources and one detector is used to increase the field of view in the axial direction. The two X-ray sources are arranged in the axial direction and alternately emit the X-rays. However, due to limitations of a cone angle and a detector dimension, an axial separation between the two X-ray sources should be set to a relatively small value, making it difficult for the axial field of view expansion capability to meet requirements, which significantly restricts the expansion capability of the field of view.

SUMMARY

The present disclosure provides a CT imaging method, an apparatus, and a CBCT device.

In a first aspect, the present disclosure provides a CT imaging method, comprising: performing several scans on an object to be scanned and obtaining one set of projection images respectively for each scan of the several scans, the several scans comprise N circular scans and M helical scans with N≥2 and N>M≥1, the several scans respectively correspond to scan layers at different axial positions, and the scan layer of the helical scans is adjacent only to the scan layer of the circular scans; performing a backprojection weighting on several projection images at the same scan angle after obtaining at least two sets of projection images, or performing the backprojection weighting on the projection images obtained at a current scan angle during each scan, to obtain weighted projection data, wherein the at least two sets of projection images comprise the projection images obtained from one circular scan and one helical scan, the scan layer of the one circular scan are axially adjacent to the scan layer of the one helical scan; and obtaining volume data of the object based on the weighted projection data.

In a second aspect, the present disclosure provides a CT imaging apparatus, comprising: a memory storing execution instructions; and a processor executing the execution instructions stored in the memory to perform the aforementioned CT imaging method.

In a third aspect, the present disclosure provides a CBCT device, comprising: an X-ray source emitting X-rays to irradiate a head of an object; a detector being arranged opposite to the X-ray source and receiving the X-rays; a support arm fixing the X-ray source and the detector, wherein the support arm, the X-ray source, and the detector form a laterally open shape, and the lateral open shape allowing the head to be inserted into the lateral open shape from a lateral direction of the CBCT device; a driving device supporting and rotating the support arm, thereby causing the X-ray source and the detector to rotate; and the aforementioned CT imaging apparatus for obtaining dental images of the object based on the X-rays received by the detector, wherein the CBCT device is configured to allow measurement in a sitting position mode or a lying/prone position mode; in the sitting position mode, the head of the object is positioned inside the lateral open shape in a sitting posture; in the lying/prone position mode, the head of the object is positioned inside the lateral opening shape in a lying/-prone posture.

BRIEF DESCRIPTION OF DRAWINGS

The drawings illustrate exemplary embodiments of the present disclosure and, together with the description, serve to explain the principles of the present disclosure. These drawings are included to provide further understanding of the present disclosure and form a part of this specification.

Fig. 1 is a flowchart schematic diagram of a CT imaging method of large field of view according to an embodiment of the present disclosure.
Fig. 2 is a flowchart schematic diagram of a method for weighting projection images according to an embodiment of the present disclosure.
Fig. 3 is a flowchart schematic diagram of a method for weighting projection images according to an embodiment of the present disclosure.
Fig. 4 is a flowchart schematic diagram of a method for weighting projection images according to an embodiment of the present disclosure.
Fig. 5 is a schematic diagram of an axial longitudinal cross-section showing effective fields of view for several circular scans.
Fig. 6 is a flowchart schematic diagram of a method for supplementing cone angle deficiency according to an embodiment of the present disclosure.
Fig. 7 is a flowchart schematic diagram of a method for supplementing cone angle deficiency according to an embodiment of the present disclosure.
Fig. 8 is a schematic diagram of a CT imaging apparatus implemented with a processing system hardware according to an embodiment of the present disclosure.
Figs. 9-17 are schematic structural diagrams of a CBCT device according to an embodiment of the present disclosure.

DETAILED DESCRIPTION

The present disclosure will be described in further detail below with reference to the drawings and embodiments. It should be understood that the specific embodiments described herein are only intended to explain relevant content and are not intended to limit the present disclosure. Additionally, for the sake of description, only parts relevant to the present disclosure are shown in the drawings.

The following description of a CT imaging method and an apparatus of a large field of view will be made with reference to the drawings, taking the application scenario of projection scan using a CBCT device as an example.

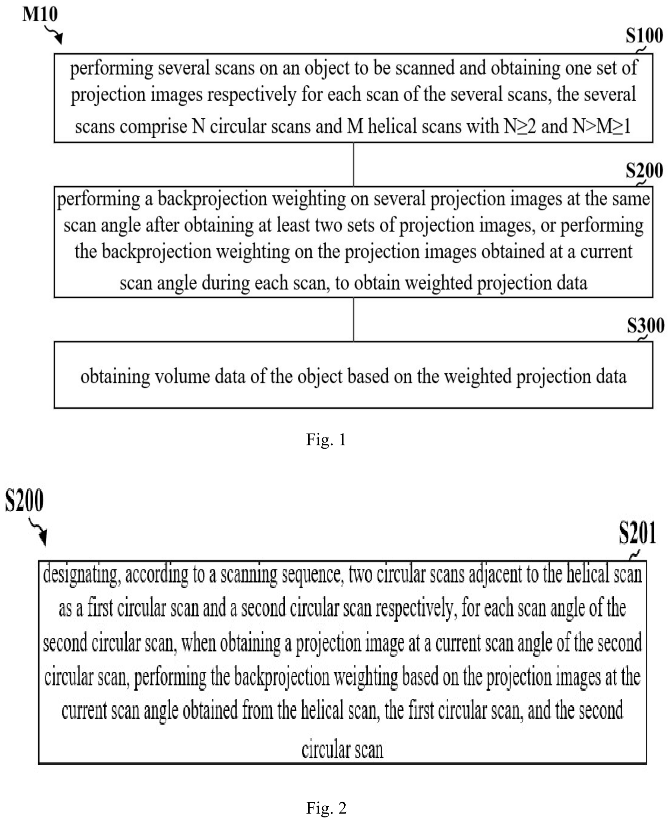

Fig. 1 is a flowchart diagram of a CT imaging method according to an embodiment of the present disclosure. The CT imaging method may be the CT imaging method of a large field-of-view. Referring to Fig. 1, the CT imaging method M10 of the embodiment may include steps S100, S200, and S300.

In the step S100, performing several scans on an object to be scanned. Each of the several scans obtains a set of projection images. The several scans include N circular scans and M helical scans, wherein N≥2 and N>M≥1. The several scans respectively correspond to scan layers at different axial positions. Each scan layers of the helical scans is adjacent only to scan layers of the circular scans.

In the step S200, performing weighted backprojection on several projection images at the same scan angle after obtaining at least two sets of projection images, or performing weighted backprojection on several projection images obtained at the current scan angle during each scanning process, to obtain weighted projection data. The at least two sets of projection images include projection images from one circular scan and one helical scan that are adjacent in the axial positions.

In the step S300, obtaining volume data of the object to be scanned based on the weighted projection data.

The scan way of the CT imaging method of the embodiment combines the circular scans and the helical scans, obtains projection data of both the circular scans and the helical scans, and performs the weighted backprojection on the obtained projection data. During the weighted backprojection process, the projection data of the helical scans are used to fill data missing in cone-angle regions of the projection data of the circular scan. The process of image reconstruction does not require compliance with a PI-line condition, and allows a larger pitch to maximize the expansion of the axial field of view. By combining the circular scans and the helical scans, the expansion of the axial field of view can be performed at any height. Therefore, the axial large field of view without cone angle missing can be obtained, and while the scan time can be reduced.

Scanning (imaging) is performed on the head or other target regions of the object to be scanned, and the scan images can be obtained. The scan images are also referred to as the projection images. One scan obtains several projection images, with several projection images obtained from the same one scan being grouped together. Several scans can obtain several groups of projection images. Within the same group of projection images, each of the projection image is obtained at a different scan angle, with the scan angle being equivalent to the imaging perspective. Different imaging perspectives result in different projection images.

The several scans include at least two circular scans and at least one helical scan. The CT imaging method M10 of the embodiment can be applied to a single-source single-detector system, with the circular scans and the helical scans being implemented using the single-source single-detector system. The single-source single-detector system includes one X-ray source and one detector.

The circular scan is also known as an axial scan. During a single circular scan, the X-ray source and the detector rotate relative to the object to be scanned in a horizontal plane perpendicular to the axial direction, with the relative positional relationship between the X-ray source and the detector remaining unchanged. The term "axial" refers to the direction of the vertical axis. The vertical axis is parallel to the ray reception area of the detector and perpendicular to the transverse section of the object to be scanned. The "axial" direction can be vertical. During a single helical scan, the X-ray source and the detector also rotate relative to the object to be scanned, with the relative positional relationship between the X-ray source and the detector remaining unchanged. However, during the rotation, the axial distance between the X-ray source and the detector and the object to be scanned changes. The rotation angle for the single helical scan does not exceed 360 degrees.

Each scan corresponds to one scan layer. The N circular scans and the M helical scans correspond to a total of N+M scan layers. Since the axial positions of the X-ray source and the detector are different during each scan, the axial positions of the scan layers are also different. The axial positions of the scan layers refer to the positions in the axial direction. The scan layers of the circular scans are briefly referred to as circular scan layers, with the axial positions of the circular scan layers being the positions of the rotation plane of the circular scans in the axial direction. The scan layers of the helical scans are briefly referred to as helical scan layers, with the axial positions of the helical scan layers being any position (e.g., the middle position) between the lowest and highest points of the scanning trajectory in the axial direction, or the midpoint position between the starting point and the ending point of the scanning trajectory in the axial direction, or other positions of the scanning trajectory in the axial direction.

Each helical scan layer is located between two circular scan layers. Assuming N=4 and M=2, the axial arrangement of all scan layers may be: a circular scan layer L1, a helical scan layer L2, a circular scan layer L3, a circular scan layer L4, a helical scan layer L5, and a circular scan layer L6. It can be understood that since no helical scan layer is provided between the circular scan layer L3 and the circular scan layer L4, the distance between the circular scan layer L3 and the circular scan layer L4 needs to be set smaller to avoid a cone angle deficiency.

During the reconstruction process using the backprojection reconstruction algorithm on projection data, the backprojection needs to be performed on projection images. The backprojection involves calculating the projection value (imaging contribution) of each projection image to every voxel in the three-dimensional target space, and adding this projection value to the CT three-dimensional voxel array. In this embodiment, the object to be scanned undergoes several scans, so at the same angle, several rays penetrate the same voxel, with these several rays corresponding to different scans at the same scan angle. Consequently, during the backprojection, weighting of the projection images is required. The weighting of the projection images normalizes these several rays, ensuring a consistent backprojection energy for each point in space, eliminating a data redundancy (excess irradiation energy) resulted from several projection images, so that only one ray penetrates each voxel at each scan angle.

The weighting can be performed using either of the following two methods. The first weighting way is that, after obtaining two or more groups of the projection images, some of which are projection images taken at the same scan angle, perform a backprojection weighting on several projection images at the same scan angle to obtain weighted projection data. The second weighting way is that, during each scan, whenever one projection image is captured, use the one projection image to perform the backprojection weighting to obtain weighted projection data. The weighted projection data obtained by the first weighting way is equivalent to the weighted projection data obtained by the second weighting way.

After obtaining the weighted projection data, the FDK (Feldkamp-Davis-Kress) reconstruction algorithm can be used to obtain three-dimensional CT image data of the object to be scanned. When using the FDK algorithm, an initial three-dimensional voxel data is formed before backprojection, with each voxel value being zero at this stage. After completing the backprojection weighting and obtaining the weighted projection data, actual voxel values in the three-dimensional voxel data are obtained, completing the reconstruction and obtaining the three-dimensional CT image data.

Exemplarily, during each scan of the several scans, the scan angle can be changed by controlling the scanning device to rotate around the object to be scanned. The head of the object to be scanned can be fixed in position using a fixing device (e.g., a head clamp). The scanning device includes the X-ray source and the detector. During scanning, the object to be scanned is positioned between the X-ray source and the detector. The X-ray source and the detector may be connected by a U-shaped connecting arm and rotate around a rotation center, forming a new scan angle and performing one imaging operation each time they rotate by one certain angle. The scanning device may also include a lifting device, with the X-ray source and the detector achieving helical lifting by controlling the lifting device and the connecting arm to rotate synchronously.

Exemplarily, during each scan of the several scans, the rotation radius of the scanning device may be the same. During the N circular scans and the M helical scans, the distance between the X-ray source and the rotation center remains unchanged, and the distance between the detector and the rotation center also remains unchanged.

Exemplarily, in the scanning device used for scanning the object to be scanned, the detector is offset-installed relative to the X-ray source. The offset means that in the line perpendicular to the detection surface of the detector and formed by connecting the X-ray source with one point of the detection surface, the intersection point of the line with the detector surface is located on one side (e.g., left or right) of the horizontal direction of the center point of the detector. For each scan of the several scans, from the start of emitting rays to initiate the scan until stopping the emission of rays to end the scan, if the detector is offset, the rotation angle of the scanning device can be 360°, thereby providing a larger field of view for horizontal scanning.

Exemplarily, during the circular scans, the axial position of the scanning device remains unchanged, while during the helical scans, the axial position of the scanning device can change unidirectionally along the axial direction. The unidirectional change means movement in only one direction along the axial direction. For example, the X-ray source and the detector synchronously rise only along the axial direction during each helical scan, or synchronously descend only along the axial direction during each helical scan.

Exemplarily, M can equal to N-1, with the scan layers of the circular scans and the helical scans alternately distributed in the axial direction. For example, with N=3 and M=2, the axial arrangement of all scan layers can be: circular scan layer L1, helical scan layer L2, circular scan layer L3, helical scan layer L4, circular scan layer L5. Exemplarily, M can equal to 1 and N can equal to 2, and the axial arrangement of all scan layers is: circular scan layer L1, helical scan layer L2, circular scan layer L3.

Exemplarily, when performing several scans on the object to be scanned, the scans are performed sequentially on the object according to the axial position distribution of the scan layers of each scan. Assuming N=2 and M=1, at the beginning of the several scans, the scanning device can first be controlled to rotate horizontally at an axial position K1 to perform the first circular scan, with the first circular scan corresponding to the circular scan layer L1 and the rotation angle of the scanning device being 360 degrees. After completing the first circular scan, the scanning device is controlled to start spiraling upward from its current position to perform the first helical scan, with the first helical scan corresponding to the helical scan layer L2 and the rotation angle of the scanning device being 360 degrees, with the axial position of the scanning device starting to rise from the axial position K1. After completing the first helical scan, the axial position of the scanning device rises to an axial position K2. The scanning device is then controlled to rotate horizontally at the axial position K2 to perform the second circular scan, with the second circular scan corresponding to the circular scan layer L3 and the rotation angle of the scanning device being 360 degrees.

It can be understood that if N>2, the process continues similarly until all scanning processes are completed.

When the projection images are weighted, the weighting of projection data can be performed after obtaining all scan projection data, or weighting can begin using the obtained projection data before completing all scans.

For the circular scans, if the rotation angle of the scanning device is less than 360 degrees, after completing the current circular scan, the next helical scan can be started directly from the position where the current circular scan was completed, or the device can rotate from the position where the current circular scan was completed to a default position without imaging during the rotation from the completion position to the default position, with the default position possibly being the starting position of the current circular scan. The next helical scan then begins from the default position.

For the helical scans, if the rotation angle of the scanning device is less than 360 degrees, after completing the current helical scan, the next circular scan can be started directly from the position where the current helical scan was completed, or it can horizontally rotate to a default position without imaging during the rotation from the completion position to the default position. The next circular scan then begins from the default position.

Exemplarily, if two adjacent scan layers correspond to different scan ways, the earlier-executed scan among the two scans corresponding to the two adjacent scan layers can be designated as a prior scan, with the other scan designated as a subsequent scan. After completing the prior scan, the scanning device can first be controlled to perform a first helical motion, and after completing the first helical motion, the subsequent scan is performed.

Continuing with the example of the scan layers L1-L5, after completing the circular scan of the circular scan layer L1, a first helical motion of a certain angle is first performed, then the helical scan of the helical scan layer L2 is started. After completing the helical scan of the helical scan layer L2, a first helical motion of a certain angle is first performed, then the circular scan of the circular scan layer L3 is started. This continues similarly, with a first helical motion of a certain angle being performed after completing the helical scan of the helical scan layer L4, then starting the circular scan of the circular scan layer L5. After completing the circular scan of the circular scan layer L5, all scanning processes are completed.

It can be understood that during the first helical motion, the scanning device does not need to perform scanning. The first helical motion is only used to achieve position movement of the scanning device.

The axial travel distance of the first helical motion can be not less than the height of the cone angle deficiency at the outermost edge of the scanning field of view of two adjacent circular scans of the subsequent scan. For example, the two first helical motions adjacent to the helical scan layer L2 are the earlier-occurring first helical motion R1 and the later-occurring first helical motion R2, with the two circular scans adjacent to helical scan layer L2 being the circular scan W1 corresponding to scan layer L1 and the circular scan W3 corresponding to scan layer L3. The axial travel distances of R1 and R2 are both not less than the height of the cone angle deficiency at the outermost edge of the scanning field of view of either scan W1 or W3, enabling the helical scan data to be more suitable for filling the cone angle deficiency portion in the circular scan data. It can be understood that since the scanning path is pre-set, information such as cone angle deficiency height during different scans can be pre-obtained based on the imaging geometry of the scanning device.

Fig. 2 is a flowchart schematic diagram of a method for weighting projection images according to an embodiment of the present disclosure. Referring to Fig. 2, in step S200, the method of performing backprojection weighting on several projection images at the same scan angle after obtaining at least two sets of projection images can include step S201.

In the step S201, according to scanning sequence, designate the two circular scans adjacent to a helical scan as a first circular scan and a second circular scan respectively. For each scan angle in the second circular scan, when obtaining a projection image at the current scan angle of the second circular scan, perform the backprojection weighting of projection images based on the projection images at the current scan angle from the helical scan, the first circular scan, and the second circular scan.

The step S201 corresponds to the first weighting way of the two weighting ways mentioned above. The second circular scan is positioned after the first circular scan in scanning sequence. After obtaining the scanning results of the first circular scan and the helical scan, the backprojection weighting begins during the second circular scan. This delayed reconstruction processing approach reduces the number of backprojection weightings, lowering the overall computation amount and computation resource occupation time of the reconstruction process. Taking N=3 and M=2 as an example, according to the axial position distribution of the three scan layers, the first circular scan (corresponding to the aforementioned first circular scan) is performed first, followed by the helical scan, and finally the second circular scan (corresponding to the aforementioned second circular scan). During the helical scan, the scanning device spirals upward. After obtaining the projection images from the helical scan and the first circular scan, the second circular scan begins. Assuming the second circular scan is set with Q scan angles (Q is the number of the scan angles), whenever the scanning device performs imaging at the current scan angle and obtains a projection image, the backprojection weighting of the projection images begins immediately, while simultaneously controlling the scanning device to rotate to the next scan angle, then beginning to capture the projection image at the next scan angle, and so on, until imaging at all Q scan angles is completed.

In a scan group formed by one helical scan and two adjacent circular scans, the backprojection weighting for this scan group is performed simultaneously when performing the last scan (i.e., the second circular scan) of the scan group, reducing the amounts of computational resources consumed and resource occupation time.

When N=2 and M=1, the aforementioned several scans performed on the object to be scanned are equivalent to one scan group. When N>2 and M=N-1, the aforementioned several scans performed on the object to be scanned are equivalent to several scan groups. Several scan groups can share the same circular scan. For example, when N=3 and M=2, there are two scan groups in total, with the last scan (the second circular scan) of the first scan group being the first scan (the first circular scan) of the second scan group. In this case, the backprojection weighting of projection images can be performed twice: the first occurs during the second circular scan of the first scan group, with the objects of backprojection weighting including the first circular scan projection images, helical scan projection images, and second circular scan projection images of the first scan group; the second occurs during the second circular scan of the second scan group, with the objects of backprojection weighting including the first circular scan projection images, helical scan projection images, and second circular scan projection images of the second scan group.

Exemplarily, during the helical scan and the first circular scan, the obtained projection images from the helical scan and the first circular scan can be cached. Whenever a projection image from the helical scan is obtained, and whenever a projection image from the first circular scan is obtained, the projection image is cached, with the projection image associated with the scan angle / number at the time of capture. During the backprojection weighting, projection images captured at the same scan angle as the current scan angle of the second circular scan are read from the cache for backprojection weighting.

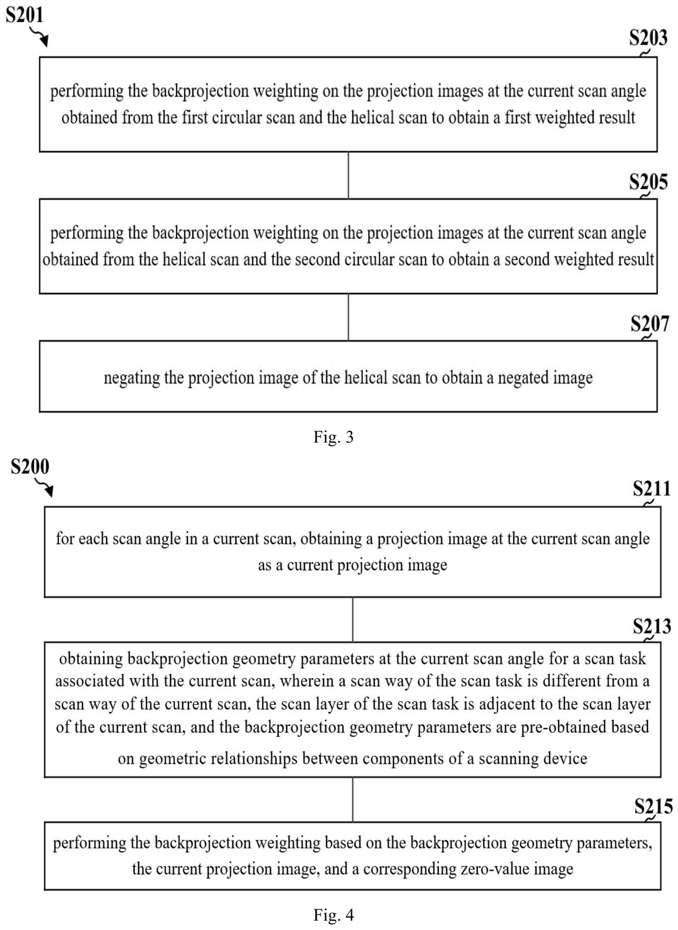

Fig. 3 is a flowchart schematic diagram of a method for weighting projection images according to another embodiment of the present disclosure. Referring to Fig. 3, step S201 can include steps S203, S205, and S207.

In the step S203, perform the backprojection weighting on projection images at the current scan angle from the first circular scan and the helical scan to obtain a first weighted result.

In the step S205, perform the backprojection weighting on projection images at the current scan angle from the helical scan and the second circular scan to obtain a second weighted result.

In the step S207, take the negative of the projection image of the helical scan to obtain a negated image.

When implementing the CT imaging method according to step S201, the first circular scan is performed first. During the first circular scan, the X-ray source and the detector horizontally rotate at the first position and perform imaging on the object to be scanned, without performing backprojection reconstruction at this time, only caching the captured projection images.

Then the helical scan is performed. During the helical scan, the X-ray source and the detector spiral upward from the first position to the second position, performing imaging on the object to be scanned during the spiral ascent, without performing backprojection reconstruction at this time, only caching the captured projection images.

Finally, the second circular scan is performed. During the second circular scan, the X-ray source and the detector horizontally rotate at the second position and perform imaging on the object to be scanned. When the X-ray source and the detector rotate to a scan angle $\omega 1$ where imaging is needed, they perform imaging and obtain the projection image C1 at the current scan angle. At this time, from the cached projection images, the projection image A1 from the first circular scan and the projection image B1 from the helical scan captured at angle $\omega 1$ are identified, then the backprojection weighting is performed on A1 and B1, as well as on B1 and C1, and the negative value image of B1 is generated, with the negative value image of B1 being -B1. The backprojection weighting fuses the two projection images, and through the results of these two backprojection weightings and the negative value image, the voxel values of the three-dimensional voxel array at the scan angle $\omega 1$ are obtained. When the X-ray source and the detector rotate to a next scan angle $\omega 2$ where imaging is needed, a projection image C2 is captured, and the images A2 and B2 are identified from the cache for backprojection weighting and taking the negative of image B2. This continues similarly for other scan angles, ultimately obtaining weighted projection data at all scan angles, which is used as actual voxel values to fill the three-dimensional voxel array, obtaining three-dimensional CT image data.

It can be understood that since during the steps S203 and S205, the projection image B1 from the helical scan participates in weighting as an object of weighting in both steps, that is to say the image B1 undergoes weighting twice, and thus the negated image of image B1 is obtained by taking its negative. In the negated image, the value at each position point is the opposite of the value at the corresponding position point on the image B1. The negated image is used to subtract the excess contribution of the image B1 from the voxels, ensuring that each projection image contributes to the voxels only once.

Fig. 4 is a flowchart schematic diagram of a method for weighting projection images according to yet another embodiment of the present disclosure. Referring to Fig. 4, in the step S200, the method of performing backprojection weighting on the projection image obtained at the current scan angle during each scan can include steps S211, S213, and S215.

In the step S211, for each scan angle in the current scan, obtain the projection image at the current scan angle as the current projection image.

In the step S213, obtain the backprojection geometry parameters at the current scan angle for scan tasks associated with the current scan, wherein the scan way of the associated scan tasks is different from the current scan and their scan layers are adjacent to the scan layer of the current scan. The backprojection geometry parameters are pre-obtained based on the geometric relationships between components in the scanning device.

In the step S215, perform the backprojection weighting based on the backprojection geometry parameters, the current projection image, and the corresponding zero-value image to obtain a weighted result. The zero-value image has the same size as the current projection image.

The steps S211, S213, and S215 correspond to the second weighting way of the two weighting ways mentioned above. Since during the first circular scan, the subsequent helical scan has not yet been performed, the backprojection weighting cannot be directly performed. Since backprojection is a linear superposition operation, the backprojection weighting can be linearly decomposed into several stages. Specifically, zero-value images can be used to replace real scan images for decomposition of weighting. Although zero-value images participate in weighting, their actual contribution is zero, serving only an auxiliary function. This allows weighting to be decomposed into two computations, distributing the computation process across each scan. Therefore, during each scan, the corresponding backprojection weighting is performed using zero-value images, reducing the storage space occupied by projection images and alleviating concentrated occupation of computation resources. Compared to the centralized weighting of the first weighting way, the second weighting way is distributed weighting.

Scan ways include helical scans and circular scans, with the helical scans and the circular scans having different scan ways, while the first circular scan and the second circular scan have the same scan way. The backprojection geometry parameters are used for calculations of backprojection weighting and are inputs to the backprojection algorithm. The backprojection geometry parameters can include the perpendicular distance from the X-ray source to the detector, the distance from the X-ray source to the rotation center, and the offset distance of the detector relative to the X-ray source. Since parameters such as scan way, rotation angle, imaging field of view, ray cone angle, physical distances between the X-ray source and the detector in the scanning device, geometric relationships, and positions during each scan are all pre-configured before actual scanning begins, the backprojection geometry parameters at each scan angle of each scan can be calculated before actual scanning begins.

The zero-value image has the same size as the corresponding projection image, with all pixel values in the image being zero. Therefore, there is no need to actually store the pixel values of each point, saving storage space.

Assuming N=2 and M=1, for the first circular scan, the associated scan tasks include only one helical scan task. The same applies to the second circular scan.

During the first circular scan, when the X-ray source and the detector rotate to a scan angle ω1 where imaging is needed, they perform imaging and obtain the projection image A1 at the current scan angle, with the image A1 being the current projection image at this time. Based on the image A1, the X-ray source-detector geometry parameters for the helical scan at the scan angle ω1 are calculated as the backprojection geometry parameters for the helical scan. Backprojection weighting is then performed based on the image A1 and the backprojection geometry parameters of the helical scan, the image A1 and the zero-value image corresponding to the image A1. This continues similarly for subsequent scan angles, obtaining the weighted results of the image A1 and the zero-value image corresponding to the image A1 at each scan angle. The zero-value image corresponding to the image A1 can be constructed immediately upon obtaining the image A1. The same applies to the second circular scan.

Exemplarily, when several scan tasks are associated with the current scan, the backprojection geometry parameters at the current scan angle are obtained separately for each associated scan task, and backprojection weighting is performed separately based on the corresponding backprojection geometry parameters, the current projection image, and the corresponding zero-value image to obtain weighted results.

Taking the helical scan as an example, assuming N=2 and M=1, the associated scan tasks include the first circular scan and the second circular scan.

During the helical scan, when the X-ray source and the detector rotate to a scan angle ω1 where imaging is needed, they perform imaging and obtain the projection image B1 at the current scan angle, with the image B1 being the current projection image at this time. Based on the image B1, the X-ray source-detector geometry parameters for the first circular scan at the scan angle ω1 are calculated as the backprojection geometry parameters for the first circular scan, and the X-ray source-detector geometry parameters for the second circular scan at the scan angle ω1 are calculated as the backprojection geometry parameters for the second circular scan. The backprojection weighting is then performed based on the image B1 and the backprojection geometry parameters of the first circular scan, the image B1 and the zero-value image corresponding to the image B1, as well as based on the image B1 and the backprojection geometry parameters of the second circular scan, the image B1 and the zero-value image corresponding to the image B1. This continues similarly for subsequent scan angles, obtaining the weighted results of the image B1 and the corresponding zero-value images at each scan angle. It can be understood that at the scan angle ω1, when the image B1 is captured, a zero-value image is used instead of the image A1 for the backprojection weighting, while when the image A1 was previously captured, a zero-value image was used instead of the image B1 for backprojection weighting. In reality, both the image A1 and the image B1 are back-projected only once.

Exemplarily, when several scan tasks are associated with the current scan, when performing the backprojection weighting based on the corresponding backprojection geometry parameters, the current projection image, and the corresponding zero-value image, the projection image of the current scan is also negated to obtain a negated image.

During the helical scan, when performing the backprojection weighting based on the image B1 and the zero-value image corresponding to the image B1, the image B1 is also negated. Since during the helical scan, the image B1 participates in weighting twice as an object of weighting, the negated image is used to subtract the excess contribution of the image B1 from the voxels, ensuring that each projection image contributes to the voxels only once.

Exemplarily, in the step S200, the method of performing the backprojection weighting on projection images can include taking spatial points that have projection points in the ray reception area during the corresponding scan angle in both scans corresponding to the two images to be backprojection weighted as first spatial points. For each first spatial point, the backprojection value of the first spatial point is calculated based on the positions of the projection points of the first spatial point in the two images and the height of the ray reception area. One of the two images is a projection image, and the other image is either a projection image or a zero-value image.

For a point P in space, if the point P has projections in several different scan projection images, this indicates data redundancy. Weighting is used to eliminate this data redundancy, ensuring that only one ray penetrates each voxel at each scan angle.

When using the first weighting way, both images being weighted are projection images. For example, one is a circular scan projection image and the other is a helical scan projection image. Since both projection images have been obtained during the backprojection weighting, having projection points in the ray reception area during the corresponding same scan angle in both scans means having projection points in both projection images. For example, the point P has projection points on the ray reception area during the same scan angle in both the first circular scan and the helical scan, indicating that the point P has projection points on both the projection image A1 and the projection image B1 at that scan angle. At this time, the point P is a first spatial point.

For the first weighting way, the specific weighting way can be to obtain the coordinate positions of the projection points of the first spatial point P in the projection images of two different scans, as well as to obtain the height of the detector's ray reception area. The backprojection value of the first spatial point P is calculated based on the obtained coordinate positions and ray reception area height. The projection point of the point P refers to the intersection point of the ray emitted by the X-ray source passing through the point P with the ray reception area of the detector. The ray reception area is the detection surface of the detector that receives rays emitted by the X-ray source, with the detection surface facing the rotation center of the scanning device and set vertically. The height of the detection surface is its length in the vertical direction. The backprojection value can be the grayscale value of the projection image after filtering (ramp filtering).

When using the second weighting way for weighting, one of the two images being weighted is a projection image, such as a circular scan projection image or a helical scan projection image, and the other is a zero-value image. The zero-value image is not an image obtained from actual scanning but serves as a substitute image replacing a real scan image, though the backprojection geometry parameters of the real image to be scanned that the zero-value image substitutes can be pre-calculated before actual scanning begins. Since actual scanning has not yet been performed, a zero-value image is used as a substitute. Therefore, although actual scanning has not yet been performed, since the geometric relationships of the scanning device are known, it can be pre-determined whether the point P will have a projection point on the ray reception area at a certain scan angle during scans that have not yet occurred.

For the second weighting way, the specific weighting way is similar to the first weighting way, obtaining the coordinate positions of the projection points of the first spatial point P in the projection image and the zero-value image, as well as obtaining the height of the ray reception area of the detector. The backprojection value of the first spatial point P is calculated based on the obtained coordinate positions and ray reception area height.

Exemplarily, the two images mentioned above include a first image A and a second image B. At least one of the image A and the image B is a projection image. The projection image is equivalent to a two-dimensional matrix. The backprojection value S of the first spatial point can be calculated using the following formula:

$$ S = \frac{A(u_a, v_a)v_a + B(u_b, v_b)(H - v_b)}{H - v_b + v_a} $$

. Where $A$

$(u_a, v_a)$ is a projection value of the projection point of the first spatial point on the first image A, $B(u_a, v_a)$ is the projection value of the projection point of the first spatial point on the second image B, with $A(u_a, v_a)v_a$ and $B(u_a, v_a)$ being grayscale values. $u_a$ and $v_a$ are the horizontal coordinate and vertical coordinate respectively of the projection point of the first spatial point on the first image A, and $u_b$ and $v_b$ are the horizontal coordinate and vertical coordinate respectively of the projection point of the first spatial point on the second image B. The upper left corner of the detector can be taken as the origin, with the direction to the right being the $u$ direction and the downward direction being the $v$ direction. $H$ is the height of the ray reception area, i.e., the height of the detection surface of the detector.

When using the first weighting way, both the first image A and the second image B are projection images. The scanning sequence of the image A can precedes that of the image B, for example, the image A is the projection image from the first circular scan, and the image B is the projection image from the helical scan.

When using the second weighting way, the first image A can be the projection image, and the second image B can be the zero-value image.

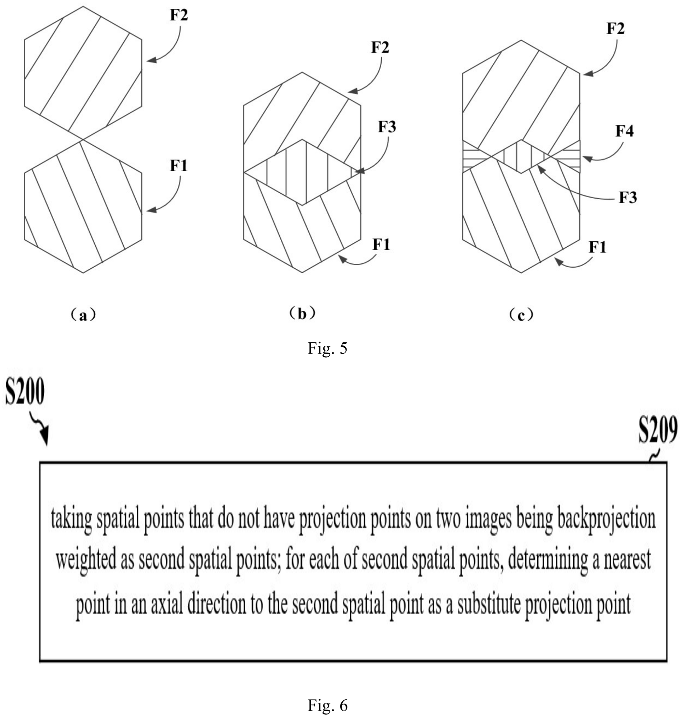

Fig. 5 is a schematic diagram of an axial longitudinal cross-section showing effective fields of view for several circular scans. Referring to Fig. 5, case (a) shows two circular scans with adjacent but non-overlapping effective fields of view, case (b) shows two circular scans with significant overlap in effective fields of view, and case (c) shows two circular scans with minimal overlap in effective fields of view.

Due to the influence of the cone beam, the effective fields of view for two circular scans are approximately spindle-shaped, meaning the upper and lower imaging boundaries are inclined. When two spindles are joined, data deficiency occurs, a situation also referred to as cone angle deficiency. The cone angle deficiency refers to the situation where some spatial points are outside the cone beam (i.e., outside the light cone formed by the detector and the X-ray source) at certain rotation angles, meaning they do not satisfy the condition of being penetrated by rays within a 180-degree range. The cone angle deficiency leads to information and energy deficiency, wherein the degree of deficiency increases as the number of missing irradiation angles for the spatial points increases.

F1 is the effective field of view of the first circular scan, and F2 is the effective field of view of the second circular scan. When performing several scans, the scan of F1 is performed first, then the scanning device is controlled to rise linearly, and then the scan of F2 is performed.

For the case (a), the scanning device rises a large distance, resulting in a large range of the cone angle deficiency between F1 and F2. If the rising distance is set even larger, the final stitched image will split in the axial direction.

For the case (b), the scanning device rises a small distance, resulting in a large overlapping F3 between F1 and F2, eliminating cone angle deficiency. However, the axial distance between F1 and F2 is small, making it difficult to meet axial field of view requirements. To meet requirements, a larger number of circular scans would be needed, resulting in lower efficiency and greater computation amount.

For the case (c), the scanning device rises a moderate distance, resulting in a small overlapping F3 between F1 and F2, but also a small range of cone angle deficiency region F4. Compared to the case (b), although the axial field of view is increased, cone angle deficiency also occurs.

If only helical scanning is used with a helical cone beam reconstruction, the PI-line condition must be satisfied, the PI-line refers to a straight line determined by any two points on the motion trajectory of the X-ray source. In practical applications, the PI-lines typically include only the line segment portion between the two trajectory points of the X-ray source. The PI-line condition can be: the point to be reconstructed is on at least one PI-line, and any light cone of the X-ray source between the two trajectory points of the X-ray source determined by this PI-line can irradiate the point to be reconstructed. The pitch is generally set to a relatively small value to ensure that PI-lines remain within the cone beam range.

Fig. 6 is a flowchart schematic diagram of a method for supplementing cone angle deficiency according to an embodiment of the present disclosure. Referring to Fig. 6, when performing backprojection weighting on projection images, the step S200 can also include step S209.

In the step S209, take spatial points that do not have projection points in both images being backprojection weighted as second spatial points. For each second spatial point, determine the nearest point in the axial direction to the second spatial point as a substitute projection point. By finding substitute projection points to fill the cone angle deficiency, the energy of the cone angle deficiency can be supplemented, ensuring that in the final reconstruction result, the cone angle deficiency region is not too dark relative to surrounding regions in terms of brightness. There are two methods for supplementing cone angle deficiency, with step S209 being the first supplementation way. The first supplementation way occurs during the backprojection weighting process.

When using the first weighting way and the first supplementation way for cone angle deficiency supplementation, when performing backprojection weighting on the first projection image A from the first circular scan and the second projection image B from the helical scan at the current scan angle $\omega 1$, for a point P in space, if the point P has projection points on both the image A and the image B, the backprojection value S is calculated; if the point P has no projection points on either the image A or the image B, it satisfies the condition for cone angle deficiency, and the point P is a spatial point in the cone angle deficiency range. At this time, the vertical coordinate $v_\alpha$ of the projection point of point P on the image A is $v_a<0$ or $v_a>H$. The nearest point in the longitudinal direction to the projection point (located outside the image) can then be taken as the projection point of the point P on the image A, i.e., by finding a substitute projection point to fill the projection point of the point P on the image A, thereby solving the cone angle deficiency problem.

When using the second weighting way and the first supplementation way for cone angle deficiency supplementation, when performing backprojection weighting on the first projection image A from the first circular scan and the corresponding zero-value image B at the current scan angle $\omega 1$, if a point P in space has no projection points on either the image A or the image B, the nearest point in the longitudinal direction to the projection point (located outside the image) can be taken as the point P's projection point on the image A, also solving the cone angle deficiency problem.

The $u_\alpha$ value of the substitute projection point remains unchanged, while $v_a$ can be set as follows: if $v_a<0$, set $v_a=0$; if $v_a>H$, set $v_a=H$. $H$ is the height of the ray reception area. The backprojection value of the point P is also the backprojection value of the substitute projection point. At this time, the substitute projection point can fall within the image A. The same applies to the image B and other points in space. Backprojection weighting and cone angle deficiency supplementation are performed together, and when obtaining the volume data of the object to be scanned in step S300, supplementation of all cone angle deficiencies is completed. It can be understood that the cone angle deficiency in Fig. 5 is the shape of the deficiency in the final reconstruction result. During the backprojection phase, cone angle deficiency manifests as a point P in deficiency space having its projection on the detector exceed the height range of the detector (essentially projection deficiency). These projection-deficient points ultimately appear on both sides when manifested in three-dimensional space.

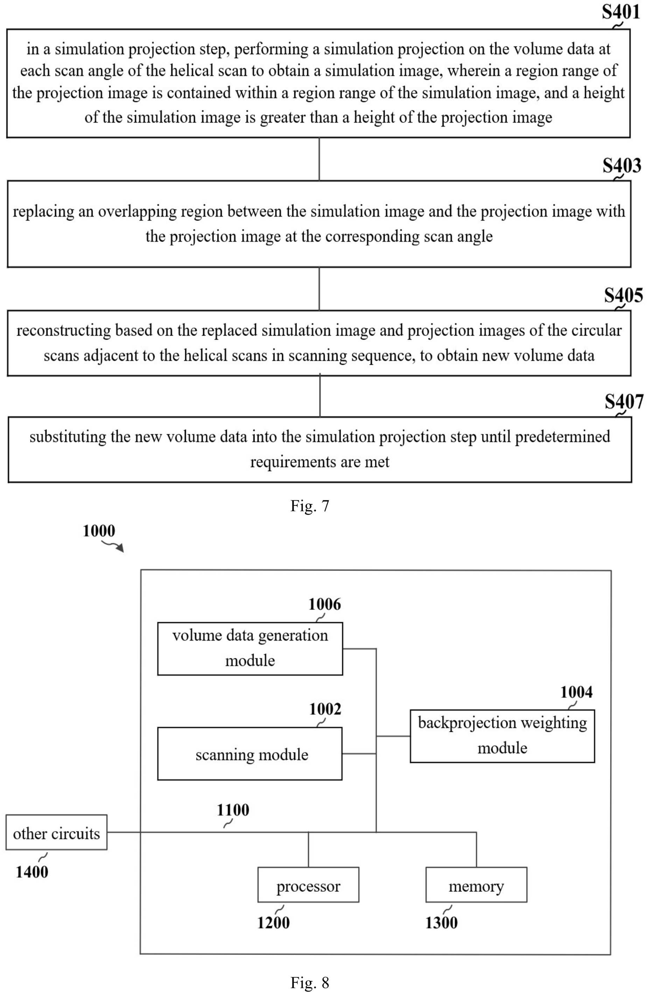

Fig. 7 is a flowchart schematic diagram of a method for supplementing cone angle deficiency according to another embodiment of the present disclosure. Referring to Fig. 7, the CT imaging method M10 can also include steps S401, S403, S405, and S407.

In the step S401 (simulation projection step), perform simulation projection on the volume data at each scan angle of the helical scan to obtain simulation images, wherein the region range of the projection image is contained within the region range of the simulation image, and the height of the simulation image is greater than the height of the projection image.

In the step S403, use the projection image at the corresponding scan angle to replace the overlapping region between the simulation image and the projection image in the simulation image.

In the step S405, reconstruct based on the replaced simulation image and the projection images of circular scans adjacent to the helical scan in scanning sequence to obtain new volume data. In the step S407, substitute the new volume data into the simulation projection step until predetermined requirements are met.

Steps S401 to S407 represent the second supplementation way for cone angle deficiency, with the second supplementation way occurring after step S300. When implementing the CT imaging method M10, both the second supplementation way and the first supplementation way can be implemented, i.e., implementing the first supplementation way during the projection process and the second supplementation way after reconstruction, or only one of the supplementation ways may be executed throughout the entire imaging method implementation process.

The second supplementation way uses the scanning results of helical scans to supplement the scan angles of cone angle deficiency, enabling points in space that originally did not satisfy the 180-degree range ray irradiation condition in circular scan projection images to receive ray supplementation, satisfying the 180-degree range ray irradiation requirement, supplementing the cone angle deficiency. Compared to the method of using the nearest point for interpolation replacement, this reduces information loss.

Simulation projection refers to using the reconstruction result (volume data) as a virtual object to be scanned for virtual scanning in a computer or device with simulation computation capability, with the obtained data being simulation projection images, briefly referred to as simulation images. The reconstruction result obtained by reconstructing real scan images is called the original reconstruction result. Since the volume data of the original reconstruction result has cone angle deficiency, during simulation projection, the virtual ray reception area must be larger than the ray reception area of the detector during real scanning, at least larger in height than during real scanning, while in length it can be larger than or equal to that during real scanning. Therefore, the simulation projection image obtained is larger in coverage range at least longitudinally compared to the real projection image, enabling the projection image obtained from one simulation projection of the reconstruction result at the same scan angle to contain more information than the projection image obtained from one real projection of the object to be scanned.

Although cone angle deficiency exists in the original reconstruction result, the cone angle deficiency region is not without any data information, but rather has incomplete data information. For example, spatial points in the cone angle deficiency region, although not penetrated by rays within a 180-degree range, are penetrated by rays within some smaller angular ranges. However, due to the small angular range, information deficiency is severe, forming artifacts. Using a larger ray reception area for simulation projection enables more abundant data information to be collected, with spatial points in the cone angle deficiency region being penetrated by rays within a larger angular range. This can supplement some ray projection data, thereby reducing artifact conditions.

Taking N=2 and M=1 as an example, CT reconstruction is performed according to steps S100, S200, and S300 to obtain reconstruction result data (three-dimensional projection image). At this time, the reconstruction result data has a certain degree of cone angle deficiency. For each projection perspective during the helical scan, simulation projection is performed using the reconstruction result data to obtain simulation images. The simulation image is a complete image with dimensions larger than the projection image at the corresponding scan angle. The region range of the real projection image at the same scan angle corresponds to the middle position region range of the simulation projection image. The simulation projection image and the original real projection image at the same scan angle are then fused. Since the simulation image completely covers the original real projection image, the overlapping region in the simulation image can be directly replaced with the original real projection image. In this way, the fused projection image has a larger longitudinal field of view coverage range, i.e., retaining the real projection image with accurate data content, while also retaining the axial expansion content formed in the simulation image before replacement. This enables the replaced simulation image to fully represent the data of the actual projection image while expanding the upper and lower boundaries.

The fused simulation image is used together with the real projection images from the first circular scan and second circular scan for reconstruction again, obtaining a new reconstruction result. The new reconstruction result is substituted into the simulation projection step for simulation projection again, obtaining simulation images at each scan angle again, then performing replacement of overlapping regions and reconstruction. This iterative process continues until predetermined requirements are met, thereby effectively supplementing the deficiency angles. The predetermined requirements can be that the number of iterations reaches a set number, or the height of the newly obtained simulation image reaches a preset height threshold, or other parameters reach their respective preset thresholds.

Fig. 8 is a schematic diagram of a CT imaging apparatus of large field of view of implemented with a processing system hardware according to an embodiment of the present disclosure. The CT imaging apparatus 1000 of this embodiment can include a memory 1300 and a processor 1200. The memory 1300 stores execution instructions, and the processor 1200 executes the execution instructions stored in the memory 1300, causing the processor 1200 to execute the CT imaging method of any of the aforementioned embodiments.

The apparatus 1000 can include corresponding modules that execute each or several steps in the aforementioned flowcharts. Therefore, each step or several steps in the aforementioned flowcharts can be executed by corresponding modules, and the apparatus can include one or more of these modules. The modules can be one or more hardware modules specifically configured to execute the corresponding steps, or implemented by processors configured to execute the corresponding steps, or stored in computer-readable media for execution by processors, or implemented through some combination.

For example, the CT imaging apparatus 1000 can include a scanning module 1002, a backprojection weighting module 1004, and a volume data generation module 1006.

The scanning module 1002 is used for performing several scans on an object to be scanned, each scan obtaining a set of projection images, with the several scans including N circular scans and M helical scans, $N{\geq}2$, $N{>}M{\geq}1$, the several scans respectively corresponding to scan layers at different axial positions, and each scan layer of the helical scans being adjacent only to scan layers of the circular scans.

The backprojection weighting module 1004 is used for, after obtaining at least two groups of projection images, performing the backprojection weighting on several projection images at the same scan angle, or during each scan, performing backprojection weighting on the projection image obtained at the current scan angle, to obtain weighted projection data, with the at least two groups of projection images including projection images from one circular scan and one helical scan whose scan layers are axially adjacent.

The volume data generation module 1006 is used for obtaining volume data of the object to be scanned based on the weighted projection data.

It should be noted that details not disclosed in the CT imaging apparatus 1000 of this embodiment can refer to the details disclosed in the CT imaging method M10 of the aforementioned embodiments of the present disclosure, and will not be repeated here.

This hardware structure can be implemented using a bus architecture. The bus architecture can include any number of interconnected buses and bridges, depending on the specific application and overall design constraints of the hardware. The bus 1100 connects various circuits including one or more processors 1200, memory 1300, and/or hardware modules. The bus 1100 can also connect various other circuits 1400 such as peripheral devices, voltage regulators, power management circuits, external antennas, etc.

The bus 1100 can be an Industry Standard Architecture (ISA) bus, Peripheral Component Interconnect (PCI) bus, or Extended Industry Standard Architecture (EISA) bus, etc. The bus can be divided into address buses, data buses, control buses, etc. For ease of representation, only one connection line is shown in the figures, but this does not mean there is only one bus or one type of bus.

Any process or method described in the flowcharts or otherwise herein can be understood as executable instruction code modules, fragments, or portions including one or more steps for implementing specific logical functions or processes. The scope of the preferred embodiments of the present disclosure includes additional implementations where the functions may not be performed in the order shown or discussed, including performing the functions in a substantially simultaneous manner or in reverse order according to the functions involved, which should be understood by those skilled in the art to which the embodiments of the present disclosure belong. The processor executes the various methods and processes described above. For example, the method embodiments of the present disclosure can be implemented as software programs tangibly contained in machine-readable media, such as memory. In some embodiments, parts or all of the software program can be loaded and/or installed via memory and/or communication interfaces. When the software program is loaded into memory and executed by the processor, one or more steps of the methods described above can be executed. Alternatively, in other embodiments, the processor can be configured to execute one of the above methods through other appropriate means (e.g., using firmware).

The logic and/or steps represented in the flowcharts or otherwise described herein can be specifically implemented in any readable storage medium for use by or in conjunction with an instruction execution system, apparatus, or device (such as a computer-based system, a system including a processor, or other systems that can fetch and execute instructions from an instruction execution system, apparatus, or device).

It should be understood that various parts of the present disclosure can be implemented using hardware, software, or combinations thereof. In the above embodiments, several steps or methods can be implemented using software stored in memory and executed by an appropriate instruction execution system. For example, if implemented using hardware, as in another embodiment, any of the following well-known techniques in the field can be used alone or in combination: discrete logic circuits with logic gate circuits for implementing logical functions of data signals, application-specific integrated circuits with appropriate combinational logic gate circuits, programmable gate arrays (PGA), field programmable gate arrays (FPGA), etc. Those of ordinary skill in the technical field can understand that all or part of the steps for implementing the method embodiments described above can be completed by instructing relevant hardware through a program, which can be stored in a readable storage medium. When executed, the program includes one or more of the steps of the method embodiments or their combinations. The storage medium can be a volatile/non-volatile storage medium. Additionally, in various embodiments of the present disclosure, functional units can be integrated into one processing module, or each unit can exist physically separately, or two or more units can be integrated into one module. The integrated modules can be implemented in hardware form or in software functional module form. If the integrated modules are implemented as software functional modules and sold or used as independent products, they can also be stored in a readable storage medium. The storage medium can be read-only memory, magnetic disk, optical disk, etc.

The present disclosure also provides a readable storage medium, with the readable storage medium storing execution instructions that, when executed by a processor, are used to implement the CT imaging method of the aforementioned embodiments.

For the purposes of this specification, a "readable storage medium" can be any device that can contain, store, communicate, propagate, or transmit a program for use by or in conjunction with an instruction execution system, apparatus, or device. More specific examples of readable storage media (non-exhaustive list) include: electrical connections (electronic devices) having one or more wires, portable computer diskettes (magnetic devices), random access memory (RAM), read-only memory (ROM), erasable programmable read-only memory (EPROM or flash memory), optical fiber devices, and portable compact disc read-only memory (CDROM). Additionally, the readable storage medium can even be paper or other suitable medium on which the program can be printed, as the program can be electronically obtained, for example, by optically scanning the paper or other medium, then editing, interpreting, or otherwise processing as necessary, and then storing it in memory.

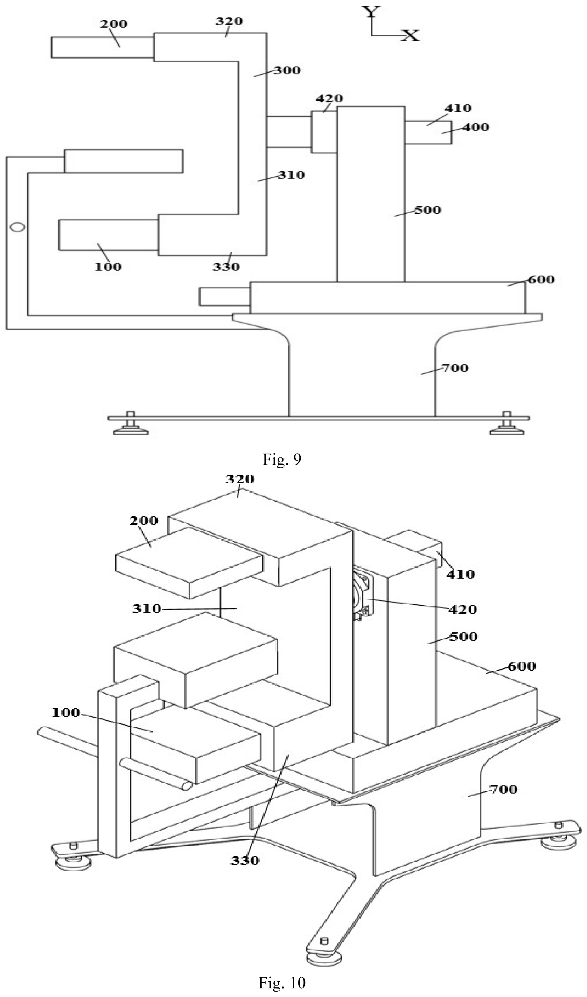

According to an embodiment of the present disclosure, a CBCT device is provided. As shown in Figs. 9 and 10, the CBCT device can include an X-ray source 100, a detector 200, a support arm 300, a driving device 400, and the aforementioned CT imaging apparatus 1000.

The X-ray source 100 can be used to emit X-rays, and the detector 200 can be used to receive X-rays. The X-ray source 100 and the detector 200 are oppositely arranged and separated by a predetermined distance. The X-ray source 100 and the detector 200 are oppositely arranged and spaced and form a receiving space that can accommodate a measurement object, such as a patient's head. In this way, the measurement object accommodated in the receiving space can be irradiated by X-rays emitted by the X-ray source 100, with the detector 200 receiving the X-rays and providing them to the aforementioned CT imaging apparatus, thereby achieving imaging of the measurement object.

The support arm 300 can be used to support and maintain the X-ray source 100 and the detector 200. The X-ray source 100 and the detector 200 can be fixed to the support arm 300. As shown in the figures, the support arm 300 can be approximately C-shaped. As an example, the support arm 300 can include a first part 310, a second part 320, and a third part 330. The first part 310 extends in the vertical direction, while the second part 320 and the third part 330 extend in the horizontal direction.

In the present disclosure, the vertical direction can be the Y direction shown in the figure, which can be perpendicular to the support surface (setting surface) on which the CBCT device is placed, or can be considered perpendicular to the plane of the base described below. The horizontal direction can be the X direction shown in the figures, which can be parallel to the support surface on which the CBCT device is placed, or can be considered parallel to the plane of the base described below. The vertical direction and horizontal direction can be intersecting directions, for example, intersecting at 90 degrees.

The first part 310, the second part 320, and the third part 330 can be integrally formed or formed by fixing together. The second part 320 and the third part 330 can be configured near the ends of the first part 310. The second part 320 can be fixed with the detector 200, and the third part 330 can be fixed with the X-ray source 100, thereby forming the vertically oppositely arranged the detector 200 and the X-ray source 100.

The driving device 400 can include a driving motor 410 and a hollow rotary platform 420. The hollow rotary platform 420 is conventional technology and will not be described in detail herein. The driving motor 410 can be used to drive the hollow rotary platform 420, with the hollow rotary platform 420 connected to the first part 310 of the support arm 300 via a connecting shaft. The second part 320 and third part 330 are configured on a first side of the first part 310, while the driving device 400 is configured on a second side of the first part 310.

In this way, the driving motor 410 of the driving device 400 drives the hollow rotary platform 420 to rotate, thereby driving the support arm 300 to rotate, ultimately achieving rotation of the X-ray source 100 and the detector 200. In the present disclosure, the rotation of the X-ray source 100 and the detector 200 is rotational motion in a plane perpendicular to the horizontal plane. The present disclosure can also include a bracket 500. The bracket 500 can be used to support the driving device 400, for example, the hollow rotary platform 420 can be installed on the bracket 500, thereby supporting the driving device 400.

The CBCT device according to the present disclosure differs from existing suspended CBCT devices and floor-standing CBCT devices. In suspended CBCT devices, a downward-opening receiving space is formed to accommodate the patient, with the rotation mechanism set above the patient's head, enabling the X-ray source and the detector to rotate on both sides of the patient's head to complete imaging. Floor-standing CBCT devices form an upward-opening receiving space to accommodate the patient, with the rotation mechanism set below the patient, positioning the X-ray source and the detector on both sides corresponding to the patient's head, enabling the X-ray source and the detector to rotate on both sides of the patient's head to complete imaging. The CBCT device of the present disclosure, however, forms a laterally opening receiving space to accommodate the patient's head. Therefore, the CBCT device of the present disclosure is clearly different in form from CBCT devices in the prior art.

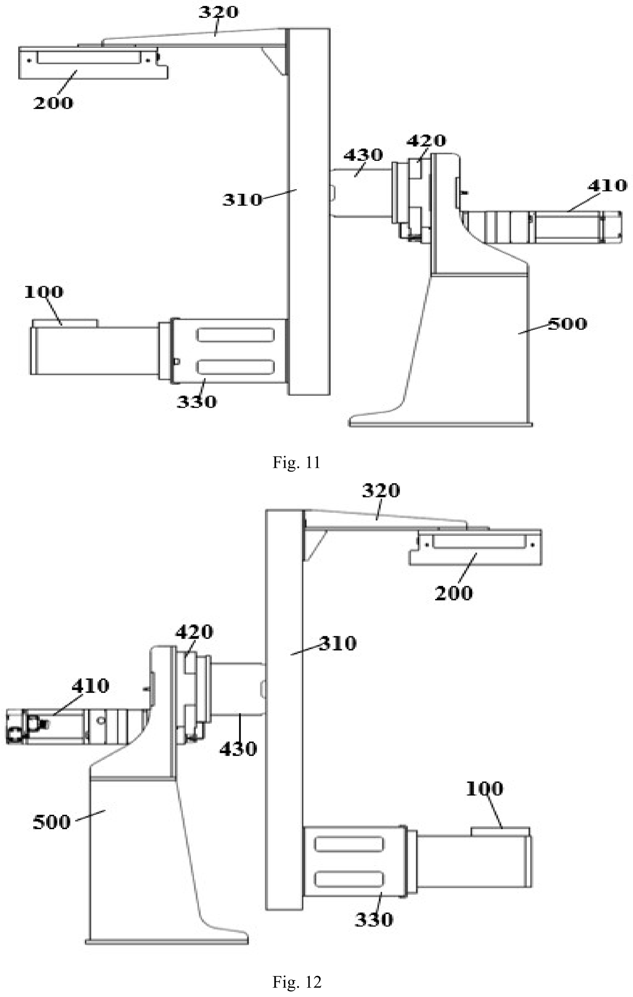

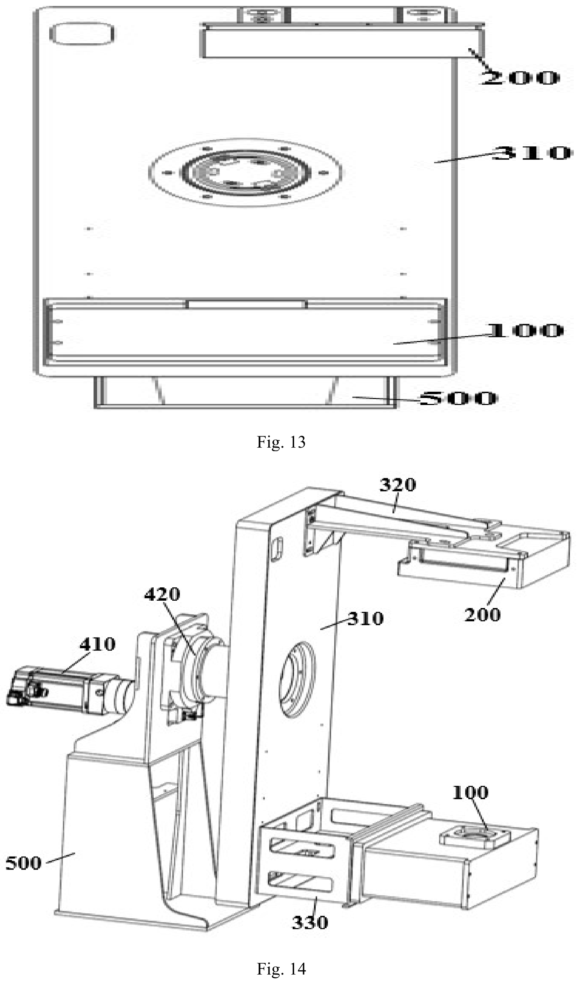

Figs. 11 to 14 show partial schematic diagrams of a CBCT device according to an embodiment of the present disclosure. The X-ray source 100 can be fixed to the first part 310 via the third part 330, with the third part 330 specifically being an X-ray source bracket. The detector 200 can be fixed to the first part 310 via the second part 320, with the second part 320 specifically being a detector bracket. The driving motor 410 can be installed on one side of the hollow rotary platform 420, while the connecting shaft 430 can be installed on the other side of the hollow rotary platform 420. An installation hole is provided on the first part 310 for installing the connecting shaft 430.

As shown in Figs. 9 and 10, the CBCT device of the present disclosure can also include a support table 600, with the bracket 500 fixedly installed on the support table 600, thereby supporting components such as the driving device, the support arm, the X-ray source, and the detector. It can also include a base 700. The base 700 can be used to fix and support the support table 600. Although the support table and base are shown in the Fig., they can be integrally formed or only the base may be provided. The upper surface of the base 700 serves as the support table.

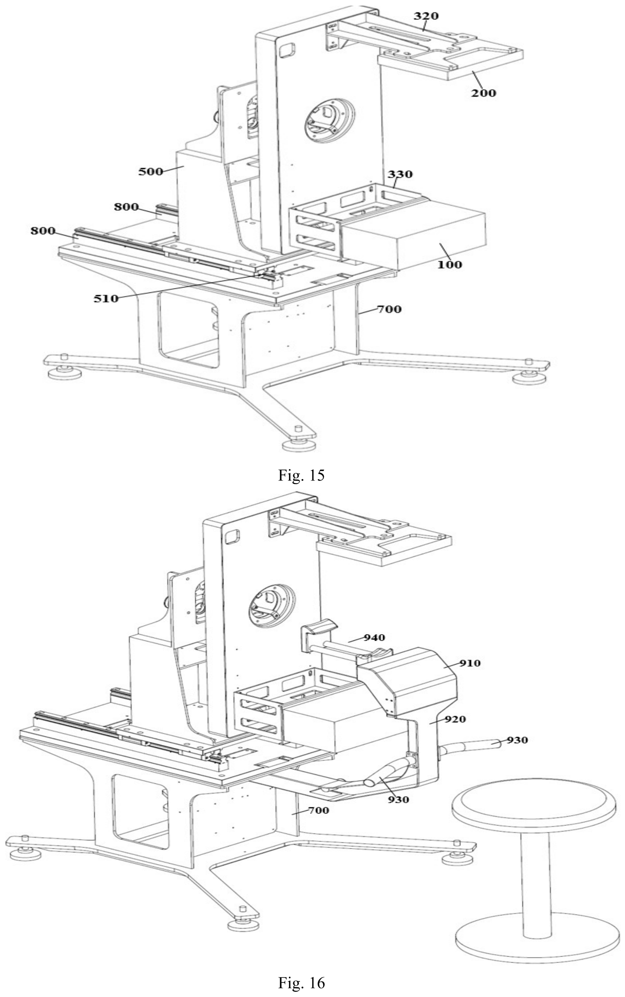

Fig. 15 shows a CBCT device according to an embodiment of the present disclosure. As shown in the figure, the bracket 500 can slide relative to the base 700 (the following will be described taking the case without a support table as an example. If a support table exists, movement and installation are relative to the support table). Guide rails 800 are fixedly provided on the base 700 (the support table). As shown in the figure, the number of guide rails can be two, separately provided on both sides of the bracket 500. The guide rails 800 extend in the horizontal direction. A sliding groove 510 can be provided on the lower part of the bracket 500, with the sliding groove 510 slidably installed on the guide rails 800, allowing the sliding groove 510 to move along the guide rails 800.

To enable the sliding groove 510 to move along the guide rails 800, a driving motor (not shown in the figures) can be provided. In this way, during imaging of the measurement object, the driving motor 410 can control the X-ray source 100 and the detector 200 to rotate in rotational motion, and the driving motor can control the X-ray source 100 and the detector 200 to move linearly. The X-ray source 100 and the detector 200 move in the horizontal plane.

The bottom of the bracket 500 can be fixed to the upper side of an installation plate 520, and the sliding groove 510 can be fixed to the lower side of the installation plate 520. Movement of the installation plate 520 drives the bracket to move, ultimately driving the X-ray source 100 and the detector 200 to move.

The CBCT device according to the present disclosure operates differently from conventional CBCT devices, proposing a new operating method. The CBCT device can laterally rotate to control the X-ray source and the detector (i.e., rotating and controlling on one side of the measurement object), with the X-ray source and the detector vertically oppositely arranged. Through this method, more space is left for the measurement object on one side, and the control of the present disclosure will be convenient and simple.

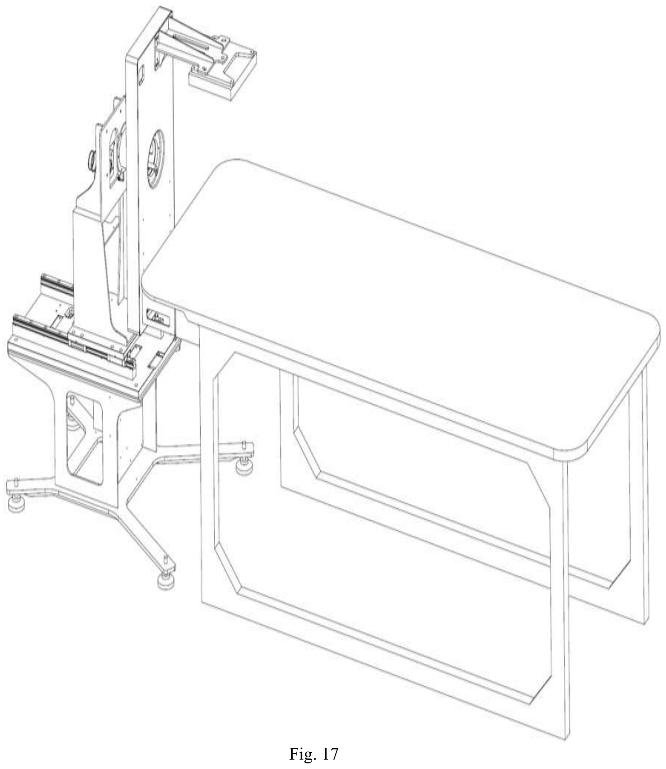

The innovatively designed CBCT device according to the present disclosure provides more diverse measurement methods. As shown in Figs. 16 and 17, the CBCT device of the present disclosure can use sitting position measurement or lying position measurement (or prone position measurement).

Fig. 16 shows a schematic diagram of the CBCT device of the present disclosure with some additional components for sitting position measurement. As shown in Fig. 16, during sitting position measurement, a chest contact component 910 can be installed. After the patient sits on a chair, their chest can rest against the chest contact component 910, thereby fixing the patient's body. The chest contact component 910 can be connected to the base 700 via a connecting component 920, thereby fixing the chest contact component 910. A handle 930 can also be provided on the connecting component 920 for the patient to hold. During sitting position measurement, a head positioning device 940 can also be included, which can be fixed to the chest contact component 910 and can support the patient's head forehead and chin to achieve positioning. In the sitting position measurement case, after the patient is positioned, the X-ray source and the detector are controlled to rotate around the patient's head. This rotation is circular rotation perpendicular to the horizontal direction, and the X-ray source and the detector can also be controlled to move in the horizontal direction.

Fig. 17 shows the CBCT device of the present disclosure used for lying position measurement (or prone position measurement). In this case, a measurement bed can be directly set corresponding to the CBCT device. The patient can lie on or prone on the measurement bed, with their head positioned between the X-ray source and the detector. After the patient is positioned, the X-ray source and the detector are controlled to rotate around the patient's head. This rotation is circular rotation perpendicular to the horizontal direction, and the X-ray source and the detector can also be controlled to move in the horizontal direction.

Those skilled in the art should understand that the above embodiments are only intended to clearly explain the present disclosure and are not intended to limit the scope of the present disclosure. For those skilled in the art, other changes or modifications can be made based on the above disclosure, and these changes or modifications remain within the scope of the present disclosure.

## Claims

1. A CT imaging method, **characterised in that** the method comprises:

   performing several scans on an object to be scanned and obtaining one set of projection images respectively for each scan of the several scans, the several scans comprise N circular scans and M helical scans with N≥2 and N>M≥1, the several scans respectively correspond to scan layers at different axial positions, and the scan layer of the helical scans is adjacent only to the scan layer of the circular scans;
   performing a backprojection weighting on several projection images at the same scan angle after obtaining at least two sets of projection images, or performing the backprojection weighting on the projection images obtained at a current scan angle during each scan, to obtain weighted projection data, wherein the at least two sets of projection images comprise the projection images obtained from one circular scan and one helical scan, the scan layer of the one circular scan are axially adjacent to the scan layer of the one helical scan; and
   obtaining volume data of the object based on the weighted projection data.

2. The CT imaging method of claim 1, **characterised in that** during each scan of the several scans, the scan angle is changed by controlling a scanning device to rotate around the object.

3. The CT imaging method of claim 1, **characterised in that** in a scanning device used for scanning the object, a detector is offset-installed relative to an X-ray source.

4. The CT imaging method of claim 1, **characterised in that** during the circular scans, an axial position of the scanning device remains unchanged, while during the helical scans, the axial position of the scanning device changes unidirectionally along an axial direction.

5. The CT imaging method of claim 1, **characterised in that** M is equal to N-1, and the scan layers of the circular scans and the scan layers of the helical scans are alternately distributed in an axial direction.

6. The CT imaging method of claim 5, **characterised in that** M is equal to 1.

7. The CT imaging method of claim 1, **characterised in that** when performing the several scans on the object, the object is scanned sequentially according to an axial position distribution of the scan layers of the serval scans.

8. The CT imaging method of claim 7, **characterised in that** when two adjacent scan layers correspond to different scan ways, an earlier-executed scan of two scans corresponding to the two adjacent scan layers is designated as a prior scan, and the other scan of the two scans is designated as a subsequent scan, after completing the prior scan, a first helical motion is performed by controlling a scanning device, and after completing the first helical motion, the subsequent scan is performed.

9. The CT imaging method of claim 8, **characterised in that** an axial travel distance of the first helical motion is not less than a height of a cone angle deficiency at an outermost edge of a scanning field of view of two adjacent circular scans of the subsequent scan.

10. The CT imaging method of claim 7, **characterised in that** performing a backprojection weighting on several projection images at the same scan angle after obtaining at least two sets of projection images comprises:
designating, according to a scanning sequence, two circular scans adjacent to the helical scan as a first circular scan and a second circular scan respectively, for each scan angle of the second circular scan, when obtaining a projection image at a current scan angle of the second circular scan, performing the backprojection weighting based on the projection images at the current scan angle obtained from the helical scan, the first circular scan, and the second circular scan.

11. The CT imaging method of claim 10, **characterised in that** during the helical scan and the first circular scan, the projection images obtained from the helical scan and the first circular scan are cached.

12. The CT imaging method of claim 10, **characterised in that** performing the backprojection weighting based on the projection images at the current scan angle obtained from the helical scan, the first circular scan, and the second circular scan comprises:

performing the backprojection weighting on the projection images at the current scan angle obtained from the first circular scan and the helical scan to obtain a first weighted result;
performing the backprojection weighting on the projection images at the current scan angle obtained from the helical scan and the second circular scan to obtain a second weighted result; and
negating the projection image of the helical scan to obtain a negated image.

13. The CT imaging method of claim 7, **characterised in that** performing the backprojection weighting on the projection images obtained at a current scan angle during each scan comprises:

for each scan angle in a current scan, obtaining a projection image at the current scan angle as a current projection image;
obtaining backprojection geometry parameters at the current scan angle for a scan task associated with the current scan, wherein a scan way of the scan task is different from a scan way of the current scan, the scan layer of the scan task is adjacent to the scan layer of the current scan, and the backprojection geometry parameters are pre-obtained based on geometric relationships between components of a scanning device; and
performing the backprojection weighting based on the backprojection geometry parameters, the current projection image, and a corresponding zero-value image having the same size as the current projection image, to obtain a weighted result.

14. The CT imaging method of claim 13, **characterised in that** when several scan tasks are associated with the current scan, the backprojection geometry parameters at the current scan angle are obtained for each scan task respectively, and the backprojection weighting is performed respectively based on the backprojection geometry parameters, the current projection image, and the corresponding zero-value image, to obtain the weighted result.

15. The CT imaging method of claim 14, **characterised in that** when the several scan tasks are associated with the current scan, during performing the backprojection weighting based on the backprojection geometry parameters, the current projection image, and the corresponding zero-value image, a negated image is also obtained by taking the negative of the current projection image of the current scan.

16. The CT imaging method of claim 1, **characterised in that** a way for performing the backprojection weighting on the projection images comprises:

taking spatial points that have projection points in a ray reception area at the corresponding scan angle in two images to be backprojection weighted as first spatial points; for each of the first spatial points, calculating a backprojection value of the first spatial point based on positions of the projection points of the first spatial point in the two images and a height of the ray reception area, wherein one of the two images is a projection image and the other image is either a projection image or a zero-value image.

17. The CT imaging method of claim 16, **characterised in that** the two images include a first image A and a second image B, and the backprojection value S of the first spatial point is calculated by the following formula:

$$S = \frac{A(u_a, v_a)v_a + B(u_b, v_b)(H - v_b)}{H - v_b + v_a},$$

where $A(u_a, v_a)$ is a projection value of a projection point of the first spatial point on the first image A, $B(u_a, v_a)$ is a projection value of a projection point of the first spatial point on the second image B, $u_a$ and $v_a$ are a horizontal coordinate and a vertical coordinate respectively of the projection point of the first spatial point on the first image A, $u_b$ and $v_b$ are a horizontal coordinate and a vertical coordinate respectively of the projection point of the first spatial point on the second image B, and H is the height of the ray reception area.

18. The CT imaging method of claim 1, **characterised in that** when performing the backprojection weighting on the projection images, the method further comprises:

taking spatial points that do not have projection points on two images being backprojection weighted as second spatial points; for each of second spatial points, determining a nearest point in an axial direction to the second spatial point as a substitute projection point.

19. The CT imaging method of claim 1, **characterised in that** after obtaining the volume data of the object, the method further comprises:

in a simulation projection step, performing a simulation projection on the volume data at each scan angle of the helical scan to obtain a simulation image, wherein a region range of the projection image is contained within a region range of the simulation image, and a height of the simulation image is greater than a height of the projection image;

replacing an overlapping region between the simulation image and the projection image with the projection image at the corresponding scan angle;

reconstructing based on the replaced simulation image and projection images of the circular scans adjacent to the helical scans in scanning sequence, to obtain new volume data; and substituting the new volume data into the simulation projection step until predetermined requirements are met.

20. A CT imaging apparatus, **characterised in that** the apparatus comprises:

a memory storing execution instructions; and a processor executing the execution instructions stored in the memory to perform the CT imaging method of claim 1.

21. A CBCT device, **characterised in that** the device comprises:

an X-ray source emitting X-rays to irradiate a head of an object;
a detector being arranged opposite to the X-ray source and receiving the X-rays;
a support arm fixing the X-ray source and the detector, wherein the support arm, the X-ray source, and the detector form a laterally open shape, and the lateral open shape allowing the head to be inserted into the lateral open shape from a lateral direction of the CBCT device;
a driving device supporting and rotating the support arm, thereby causing the X-ray source and the detector to rotate; and
the CT imaging apparatus of claim 20 for obtaining dental images of the object based on the X-rays received by the detector,
wherein the CBCT device is configured to allow measurement in a sitting position mode or a lying/prone position mode; in the sitting position mode, the head of the object is positioned inside the lateral open shape in a sitting posture; in the lying/prone position mode, the head of the object is positioned inside the lateral opening shape in a lying/prone posture.

22. The CBCT device of claim 21, **characterised in that** the device further comprises:

a bracket supporting the driving device; and a base supporting the bracket, wherein the bracket is slidable relative to the base.

23. The CBCT device of claim 22, **characterised in that** the bracket is slidable relative to the base along a lateral direction of the CBCT device, and the X-ray source and the detector are controlled to perform simultaneously a rotational motion and a linear movement.

**M10**

**S100**

performing several scans on an object to be scanned and obtaining one set of projection images respectively for each scan of the several scans, the several scans comprise N circular scans and M helical scans with N≥2 and N>M≥1

**S200**

performing a backprojection weighting on several projection images at the same scan angle after obtaining at least two sets of projection images, or performing the backprojection weighting on the projection images obtained at a current scan angle during each scan, to obtain weighted projection data

**S300**

obtaining volume data of the object based on the weighted projection data

Fig. 1

**S200**

**S201**

designating, according to a scanning sequence, two circular scans adjacent to the helical scan as a first circular scan and a second circular scan respectively, for each scan angle of the second circular scan, when obtaining a projection image at a current scan angle of the second circular scan, performing the backprojection weighting based on the projection images at the current scan angle obtained from the helical scan, the first circular scan, and the second circular scan

Fig. 2

S201

S203

performing the backprojection weighting on the projection images at the current scan angle obtained from the first circular scan and the helical scan to obtain a first weighted result

S205

performing the backprojection weighting on the projection images at the current scan angle obtained from the helical scan and the second circular scan to obtain a second weighted result

S207

negating the projection image of the helical scan to obtain a negated image

Fig. 3

S200

S211

for each scan angle in a current scan, obtaining a projection image at the current scan angle as a current projection image

S213

obtaining backprojection geometry parameters at the current scan angle for a scan task associated with the current scan, wherein a scan way of the scan task is different from a scan way of the current scan, the scan layer of the scan task is adjacent to the scan layer of the current scan, and the backprojection geometry parameters are pre-obtained based on geometric relationships between components of a scanning device

S215

performing the backprojection weighting based on the backprojection geometry parameters, the current projection image, and a corresponding zero-value image

Fig. 4

**F2**
**F1**

（a）

**F2**
**F3**
**F1**

（b）

**F2**
**F4**
**F3**
**F1**

（c）

Fig. 5

**S200**

**S209**

taking spatial points that do not have projection points on two images being backprojection weighted as second spatial points; for each of second spatial points, determining a nearest point in an axial direction to the second spatial point as a substitute projection point

Fig. 6

**S401**

in a simulation projection step, performing a simulation projection on the volume data at each scan angle of the helical scan to obtain a simulation image, wherein a region range of the projection image is contained within a region range of the simulation image, and a height of the simulation image is greater than a height of the projection image

**S403**

replacing an overlapping region between the simulation image and the projection image with the projection image at the corresponding scan angle

**S405**

reconstructing based on the replaced simulation image and projection images of the circular scans adjacent to the helical scans in scanning sequence, to obtain new volume data

**S407**

substituting the new volume data into the simulation projection step until predetermined requirements are met

Fig. 7

**1000**

volume data generation module **1006**

scanning module **1002**

backprojection weighting module **1004**

other circuits **1400**

**1100**

processor **1200**

memory **1300**

Fig. 8

Fig. 9

Fig. 10

Fig. 11

Fig. 12

Fig. 13

Fig. 14

Fig. 15

Fig. 16

Fig. 17

**EP 4 742 163 A1**

## INTERNATIONAL SEARCH REPORT

| International application No. |
|---|
| **PCT/CN2023/140100** |

**A. CLASSIFICATION OF SUBJECT MATTER**

G06T11/00(2006.01)i; G06T3/00(2024.01)i; A61B6/00(2024.01)i

According to International Patent Classification (IPC) or to both national classification and IPC

**B. FIELDS SEARCHED**

Minimum documentation searched (classification system followed by classification symbols)

IPC: G06T, A61B

Documentation searched other than minimum documentation to the extent that such documents are included in the fields searched

Electronic data base consulted during the international search (name of data base and, where practicable, search terms used)

CNTXT, ENTXT, ENTXTC, DWPI, CNKI: CT, 成像, 扫描, 环形, 螺旋, 层, 角度, 权, 投影, imaging, computed tomography, scan, annular, spiral, projection, angle, weight

**C. DOCUMENTS CONSIDERED TO BE RELEVANT**

| Category* | Citation of document, with indication, where appropriate, of the relevant passages | Relevant to claim No. |
|---|---|---|
| PX | CN 116543071 A (YOFO (HEFEI) MEDICAL TECHNOLOGY CO., LTD.) 04 August 2023 (2023-08-04) entire document | 1-20 |
| PY | CN 116543071 A (YOFO (HEFEI) MEDICAL TECHNOLOGY CO., LTD.) 04 August 2023 (2023-08-04) entire document | 21-23 |
| PY | CN 116831613 A (YOFO (HEFEI) MEDICAL TECHNOLOGY CO., LTD.) 03 October 2023 (2023-10-03) entire document | 21-23 |
| A | CN 102727188 A (INSTITUTE OF AUTOMATION, CHINESE ACADEMY OF SCIENCES) 17 October 2012 (2012-10-17) entire document | 1-23 |
| A | CN 103679768 A (SIEMENS AG) 26 March 2014 (2014-03-26) entire document | 1-23 |
| A | EP 0969414 A2 (GENERAL ELECTRIC COMPANY) 05 January 2000 (2000-01-05) entire document | 1-23 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **26 January 2024** | **02 February 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

27

**EP 4 742 163 A1**

**INTERNATIONAL SEARCH REPORT**

| International application No. |
| --- |
| **PCT/CN2023/140100** |

**C.     DOCUMENTS CONSIDERED TO BE RELEVANT**

| Category* | Citation of document, with indication, where appropriate, of the relevant passages | Relevant to claim No. |
| --- | --- | --- |
| A | US 8031828 B1 (GENERAL ELECTRIC COMPANY) 04 October 2011 (2011-10-04)<br>entire document | 1-23 |

Form PCT/ISA/210 (second sheet) (July 2022)

28

**INTERNATIONAL SEARCH REPORT**
Information on patent family members

International application No.

**PCT/CN2023/140100**

| Patent document cited in search report | | | Publication date (day/month/year) | Patent family member(s) | | | Publication date (day/month/year) |
|---|---|---|---|---|---|---|---|
| CN | 116543071 | A | 04 August 2023 | CN | 116543071 | B | 19 September 2023 |
| CN | 116831613 | A | 03 October 2023 | None | | | |
| CN | 102727188 | A | 17 October 2012 | CN | 102727188 | B | 18 February 2015 |
| CN | 103679768 | A | 26 March 2014 | DE | 102012217163 | A1 | 12 June 2014 |
| | | | | DE | 102012217163 | B4 | 02 June 2022 |
| | | | | US | 2014086466 | A1 | 27 March 2014 |
| | | | | US | 9299140 | B2 | 29 March 2016 |
| | | | | CN | 103679768 | B | 12 April 2017 |
| EP | 0969414 | A2 | 05 January 2000 | EP | 0969414 | A3 | 29 January 2003 |
| | | | | EP | 0969414 | B1 | 27 September 2006 |
| | | | | JP | 2000139900 | A | 23 May 2000 |
| | | | | JP | 4558858 | B2 | 06 October 2010 |
| | | | | IL | 130718 | A0 | 01 June 2000 |
| | | | | IL | 130718 | A | 28 March 2004 |
| | | | | US | 6038278 | A | 14 March 2000 |
| | | | | DE | 69933338 | D1 | 09 November 2006 |
| | | | | DE | 69933338 | T2 | 12 April 2007 |
| US | 8031828 | B1 | 04 October 2011 | EP | 2383702 | A1 | 02 November 2011 |
| | | | | JP | 2011235088 | A | 24 November 2011 |
| | | | | JP | 5802044 | B2 | 28 October 2015 |

Form PCT/ISA/210 (patent family annex) (July 2022)